# EUROPEAN PATENT APPLICATION

(11) **EP 4 085 895 A1**
(43) Date of publication of application: **09.11.2022**
(21) Application number: 20909089.3
(22) Date of filing: 30.12.2020
(51) Int. Cl.: A61K 8/9717, A61K 8/60, A61K 8/44, A61Q 17/04, A61K 36/04, A61K 31/7028, A61K 31/7052, A61K 31/198, A61P 17/16

(54) **UV BLOCKING COMPOSITION COMPRISING RED ALGAE-DERIVED FLORIDOSIDE AND AMINE GROUP-CONTAINING COMPOUND**

(30) Priority: 30.12.2019 KR 20190178205
(71) Applicant: Athena Co., Ltd., Gyeonggi-do 16226 (KR)
(72) Inventor: PARK, Eunkyung, Yongin-si Gyeonggi-do 16849 (KR); WOO, Dongjin, Yongin-si Gyeonggi-do 16849 (KR)
(74) Representative: Eisenführ Speiser
(86) International application number: PCT/KR2020/019467
(87) International publication number: WO 2021/137647

(57) **Abstract**

The present invention relates to a UV blocking composition comprising red algae-derived floridoside and an amine group-containing compound, and a use thereof in a cosmetic material and a pharmaceutical composition.

## Description

### Technical Field

The present disclosure relates to a UV blocking composition containing red algae-derived floridoside and an amine group-containing compound and, more specifically, to a UV blocking composition containing floridoside, such as glycerol galactoside or glycerol glucoside, and a DNA or RNA nucleic acid, especially, a constituent of a nucleic acid, such as a nucleobase (a purine or a pyrimidine), a nucleoside (a ribonucleoside or a deoxyribonucleoside), or an amino acid.

### Background Art

Ultraviolet rays are divided into long-wavelength ultraviolet rays (UV A, 320-400 nm), medium-wavelength ultraviolet rays (UV B, 280-320 nm), and short-wavelength ultraviolet rays (UV C, 200-280 nm) according to the wavelength region thereof. The ultraviolet rays may be a leading cause of generation of freckles or blemish by accelerating the formation of erythema or the generation of melanin inside skin cells, or may cause skin troubles by reacting with sebum secreted from the epidermis to generate peroxide lipids, and even in serious cases, to cause skin cancer.

Blue light has a wavelength of 380-500 nm in the visible light, and is emitted in large quantities from displays and lighting devices, such as smartphones, computers, and TVs, and the repeated exposure to blue light over a long period of time may increase reactive oxygen species to induce premature aging of the skin as well as various types of skin diseases.

In the rays of the sun, short-wavelength UV C is absorbed and scattered in the ozone layer and the atmosphere and thus does not reach the surface of the earth, but approximately 6% of UV A and UV B reaches the surface of the earth, and these UV rays may be beneficial due to vitamin D synthesis in the body, treatment of skin diseases, sterilization effects, and the like, but may be harmful, for example, causing sunburn, skin aging, photosensitive skin diseases, skin cancer, and mutagenesis. In particular, UV A penetrates into the dermis layer to mainly cause pigmentation and skin aging and is involved in the occurrence of inflammatory responses and photo-sensitive skin diseases. Therefore, there is an urgent need for the development of natural materials that neither have photo-toxicity nor causes allergy and can effectively block UV A and UV B.

Titanium dioxide and zinc oxide conventionally used as UV scattering agents are difficult to disperse and cause skin whitening. Higher contents of titanium dioxide and zinc oxide give stronger irritation to the skin. Moreover, most of the synthetic UV organic absorbers that are generally often used are compounds having a conjugated double bond in the molecule, and when the molecule receives UV, electrons in the molecule have a raised energy level, resulting in an excited state, and thus are unstable, and thus the actual efficacy of the UV organic absorbers is difficult to maintain, and the molecule absorbing UV light emit various forms of energy when returning to the ground state, causing side effects, such as skin irritation, heat generation, and light emission.

Flavonoid pigments, such as gallic acid and caffeic acid, which are involved in the UV blocking mechanisms of land plants, and aromatic compounds, such as, phenylalanine, tryptophan, and purines, are being studied as naturally derived UV blockers. For example, Korean Patent Publication No. 10-1995-0012443 discloses a cosmetic material containing an herbal medicine extract with UV absorption performance.

In addition, mycosporine-like amino acids (MAAs) derived from marine plants or microalgae are known to have a blocking mechanism against UV and thus are being actively studied. For example, Korean Patent Publication No. 10-2013-0081410 discloses a UV blocking composition applied externally to the skin, the composition containing a microalgae extract-derived peptide derivative.

However, these have limitations in industrial uses since the correlations between structures and UV absorption performance ranges of naturally derived UV blocking ingredient candidate groups, intensities thereof, and the like have not been yet studied.

Therefore, there is an urgent need to develop natural UV blockers that are harmless to the human body, have no side effects, are stable, are easy to use, and acts within a wide UV range of 280-400 nm.

### (PRIOR ART DOCUMENTS)

Korean Patent Publication No. 10-1995-0012443
Korean Patent Publication No. 10-2013-0081410

### Disclosure of Invention

### Technical Problem

The present inventors endeavored to develop a natural UV blocker, which has UV absorption performance without causing skin whitening and skin irritation, which are drawbacks of inorganic UV scatters among conventional UV blocking materials, and without causing side effects of photo-toxicity, skin irritation, heat generation, and light emission, which are drawbacks of organic UV absorbents, is harmless to the human body, has no side effects, and is stable and easy to use in an aspect of formulation. As a result, the present inventors discovered that a composition obtained by mixing or combining a specific material derived from red algae and an amine group-containing compound exhibits UV absorption performance in a broad range of 280-400 nm, and identified that this composition can be used as a UV blocker, and thus completed the present disclosure.

### Solution to Problem

In accordance with an aspect of the present disclosure, there is provided a UV blocking composition containing red algae-derived floridoside and an amine group-containing compound.

In accordance with another aspect of the present disclosure, there is provided a UV blocking cosmetic composition containing the composition.

In accordance with still another aspect of the present disclosure, there is provided a UV blocking pharmaceutical composition containing the composition.

### Advantageous Effects of Invention

The composition according to the present disclosure exhibits an excellent effect in blocking and preventing UV radiation in a broad range of 280-400 nm. The composition according to the present disclosure does not cause skin whitening and skin irritation, which are drawbacks of inorganic UV scatters among conventional UV blocking materials, and side effects of photo-toxicity, skin irritation, heat generation, and light emission, which are drawbacks of organic UV absorbents, and thus the composition can be advantageously used as an active ingredient for compositions for cosmetic products, medical products, and the like, for UV blocking and prevention.

### Brief Description of Drawings

FIG. 1 is a graph obtained by measurement of UV absorption performance of floridoside according to an embodiment of the present invention.
FIG. 2 illustrates a ¹H-NMR graph of floridoside according to an embodiment of the present disclosure.
FIG. 3 illustrates a ¹³C-NMR graph of floridoside according to an embodiment of the present disclosure.

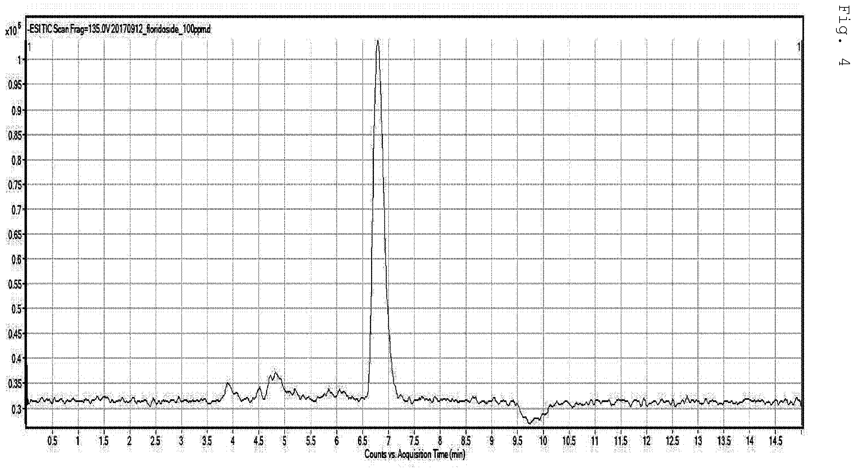

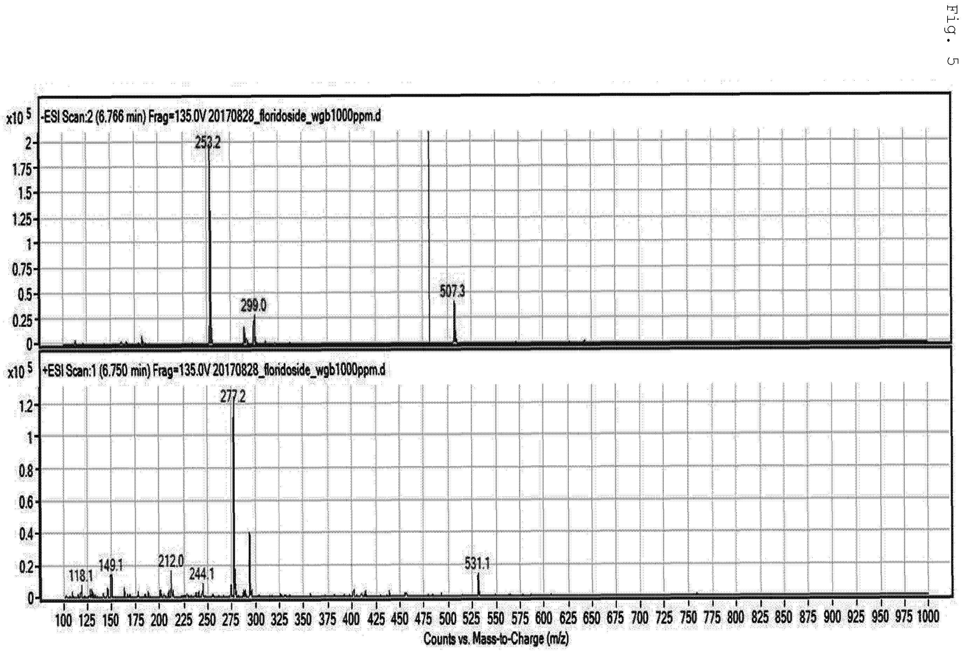

FIGS. 4 and 5 illustrate mass spectrum graphs of floridoside according to an embodiment of the present disclosure.
FIG. 6 is a graph obtained by measurement of UV absorption performance of a glycoside covalent derivative of floridoside and a nucleobase according to an embodiment of the present disclosure.
FIG. 7 is a graph obtained by measurement of UV absorption performance of a glycoside covalent derivative of floridoside and a nucleoside according to an embodiment of the present disclosure.
FIGS. 8 and 9 are graphs obtained by measurement of UV absorption performance of a glycoside covalent derivative of floridoside and an amino acid according to an embodiment of the present disclosure.

### Best Mode for Carrying out the Invention

Hereinafter, the present disclosure will be described in detail.

An embodiment of the present disclosure is directed to a UV blocking composition containing red algae-derived floridoside and an amine group-containing compound.

In the present disclosure, the red algae may include the class Bangiophyceae, the class Compsopogonophyceae, or the class Florideophyceae. The class Bangiophyceae preferably includes the order Bangiales, but is not particularly limited thereto. The class Florideophyceae preferably includes the order Ceramiales, the order Corallinales, the order Gelidiales, the order Gracilariales, the order Halymeniale, the order Nemaliales, the order Nemastomatales, the order Palmariales, or the order Stylonematales, but is not particularly limited thereto.

The floridoside is a low-molecular weight carbohydrate stored in cells of red alga and a kind of galactose glycoside. Floridoside has an average mass of 254.234 Da, a monoisotopic mass of 254.100174 Da, and a molecular formula of C₉H₁₈O₈. Floridoside is also called 2-O-glycerol-α-D-galactopyranoside or 2-*O*-*α*-D-galactopyranosylglycerol.

According to an embodiment of the present disclosure, the red algae-derived floridoside may be red algae-derived glycerol galactoside and/or red algae-derived glycerol glucoside.

A galactoside is a glycoside having galactose as a sugar component and is a compound in which hydrogen of the hydroxy group on carbon-1 of galactose is replaced by an organic moiety. There are α-galactosides and β-galactosides according to the bonding configuration.

The glycerol galactoside may be floridoside represented by Chemical Formula 1, or a salt or isomer thereof.

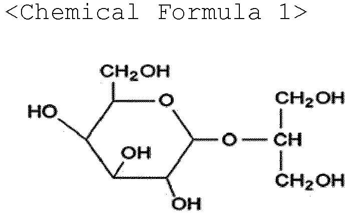

Alternatively, the glycerol galactoside may be isofloridoside represented by Chemical Formula 2, or a salt or isomer thereof.

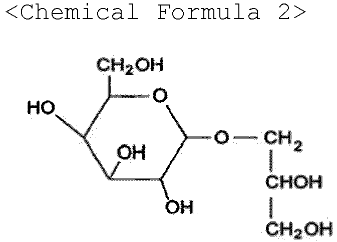

The isofloridoside is also called 1-O-glycerol-α-D-galactopyranoside.

The floridoside and isofloridoside are preferably derived from red algae.

According to an embodiment of the present disclosure, the UV blocking composition may be one in which red algae-derived floridoside is (covalently) bound or mixed with an amine group-containing compound.

Specifically, the UV blocking composition may be one in which red algae-derived floridoside is alkylated by an amine group-containing compound.

The amine group-containing compound may be at least one selected from the group consisting of a nucleobase, a nucleoside, and an amino acid.

The nucleobase may be at least one selected from adenine, guanine, cytosine, thymine, and uracil. In addition, the nucleoside may be a ribonucleoside or a deoxyribonucleoside.

An embodiment of the present disclosure is directed to a composition formed by covalently binding or mixing red algae-derived floridoside and an amine group-containing compound, and exhibits an excellent UV blocking and preventing effect.

Preferably, the UV blocking composition includes a glycoside covalent derivative of red algae-derived floridoside and an amine group-containing compound.

According to an embodiment of the present disclosure, the UV blocking composition may include a glycoside covalent derivative of red algae-derived floridoside and a nucleobase.

According to another embodiment of the present disclosure, the UV blocking composition may include a glycoside covalent derivative of red algae-derived floridoside and a nucleoside.

According to still another embodiment of the present disclosure, the UV blocking composition may include a glycoside covalent derivative of red algae-derived floridoside and an amino acid.

A UV blocking cosmetic composition according to an embodiment of the present disclosure has UV absorption performance and is surprisingly found to have excellent ability to absorb wavelengths over a wide UV region of 280-400 nm including both UV B (290~320nm) and UV A(320~400nm).

Therefore, the cosmetic composition according to the embodiment of the present disclosure may be used as a naturally derived UV blocker or as a composition that helps UV absorption.

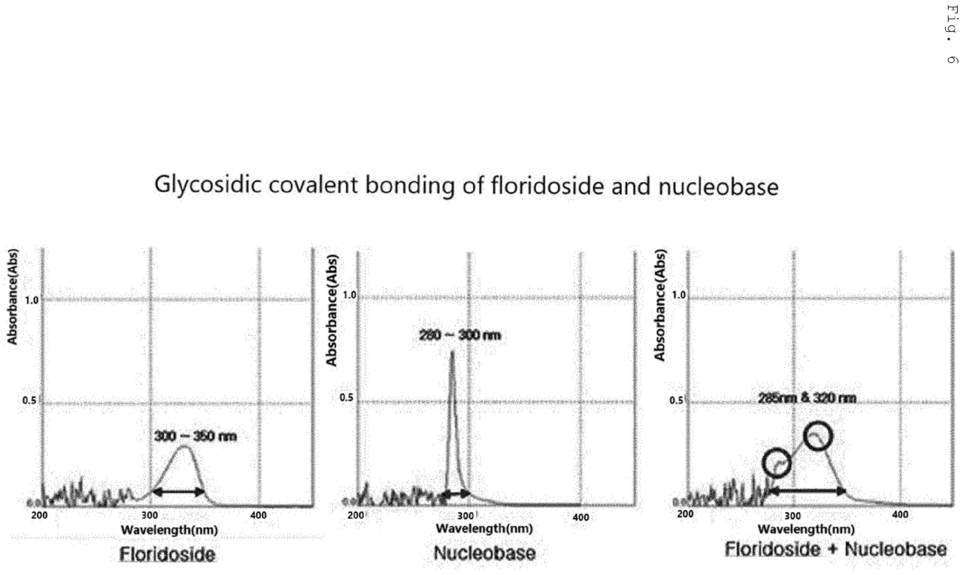

In particular, a covalent derivative of red algae-derived floridoside and a nucleobase, a nucleoside, or an amino acid, which are contained in a composition according to an embodiment of the present disclosure, is well dissolved in water, safe against heat and light, free from toxicity to cause no irritation to the skin, and exhibits a feature of absorbing a UV range of 280-400 nm, leading to a remarkable effect in blocking UV A and B. As shown in FIGS. 6 to 9, the glycoside covalent derivatives of red algae-derived floridoside and a nucleobase, a nucleoside, or an amino acid had excellent ability to absorb wavelengths over the broad UV entire range of 280-400 nm.

In an embodiment of the present disclosure, the UV blocking cosmetic composition preferably contains 1-30 wt% of the glycoside covalent derivative of red algae-derived floridoside and a nucleobase, a nucleoside, or an amino acid.

A UV blocking cosmetic composition according to an embodiment of the present disclosure preferably blocks blue light. The blue light blocking effect is particularly meaningful since blue light penetrates to the deepest site of the skin.

### Mode for Carrying out the Invention

Hereinafter, the present invention will be described in more detail with reference to exemplary embodiments. These exemplary embodiments are provided only for the purpose of illustrating the present disclosure in more detail, and therefore, according to the purpose of the present disclosure, it would be apparent to a person skilled in the art that these examples are not construed to limit the scope of the present disclosure.

### Preparative Example 1: Preparation of Floridoside

*Gracilaria vermiculophylla* (a kind of red algae) was collected in Jangheung and Wando island, South Jeolla Province, Korea, and then subjected to room-temperature extraction using a mixture solvent containing ethanol and methanol for 24 hours, followed by hot-water extraction for 6 hours. The extract was concentrated by removing salts and polysaccharides through filtration, and subjected to repeated fractionation and purification to obtain 95% or more of a floridoside powder of 254 Dal, which was then diluted with distilled water.

The obtained material was purified through the HILIC column, thereby finally obtaining a sample with 99% or more of floridoside. To investigate whether the obtained sample was floridoside, structure analysis was performed using the following materials and reagents.
Preparative-liquid chromatography (prep-LC) (LC-Forte/R, YMC, Japan)
Venusil Hilic (21.2 × 250 mm, 5 □m, Agela, USA)
VisionHT Hilic (4.6 × 250 mm, 5 □m, Grace, USA)
Kiesel gel 60F254 plate (Merck Co., Darmastdt, Germany)
RP-18 F254s plates (Merck Co., Darmastdt, Germany)
Bruker AVANCE II 400 (1H NMR at 400 MHz, 13C NMR at 100 MHz)
6530 Accurate-Mass Triple quald. LC-MS/MS (Agilent Technologies, Germany)

All the compounds used for structure analysis were purchased from Sigma Chemical Co. (St. Louis, MO, USA).

### NMR Structure Analysis

Hydrogen-related chemical environments were interpreted from chemical shifts on the ¹H-NMR spectrum; three-dimensional configurations with neighboring hydrogen atoms were interpreted from coupling constants; the number of hydrogen atoms was identified from integrations; and the number of carbon atoms constituting a compound and neighboring environments of the carbon atoms were interpreted from the ¹³C-NMR spectrum.

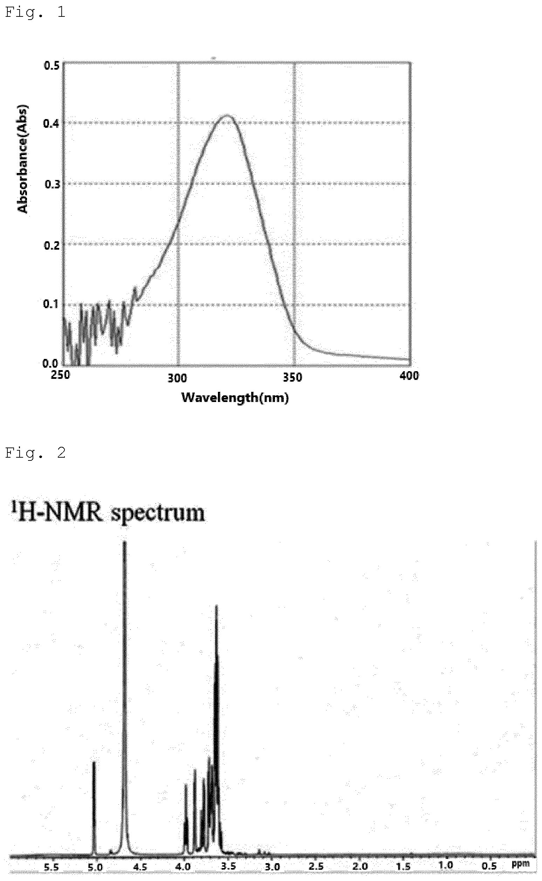

As can be identified from FIG. 2, the anomer proton signal (δ_{H} 5.03, d, J = 4.0 Hz, H-1) resulting from the sugar, oxygenated methine proton signals shown as a sugar moiety, and oxygenated methylene proton signals (δ_{H} 3.60-3.99, m, H-2, 3, 4, 5, and 6) were observed in the ¹H-NMR spectrum, and this was predicted to be glucose or galactose.

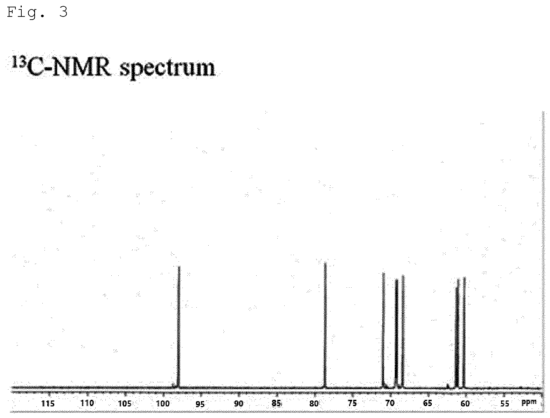

As can be identified from FIG. 3, the presence of glycerol composed of three carbon atoms, including one molecule of sugar, was identified through a total of nine carbon atoms from the ¹³C-NMR spectrum. The sugar was identified to be galactose through the anomer carbon signal δ_{c} 98.03 (C-1) resulting from the sugar, oxygenated methine carbon signals [δ_{c} 71.03 (C-5), 69.32 (C-3), 69.21 (C-4) and 68.44 (C-2)] and oxygenated methylene carbon signal δ_{c} 61.10 (C-6) .

The presence of glycerol was identified through one oxygenated methine carbon signal δ_{c} 78.68 (C-2') and two oxygenated methylene carbon signals [δ_{c} 61.35 (C-1') and 60.31 (C-3')]. Therefore, the above-mentioned compound (sample) was identified to have a structure of 2-*O*-*α*-D-galactopyranosylglycerol (floridoside).

As a result of ESI-MS measurement, 253 [M-H]⁻ in the negative mode and 277 [M+Na]⁺ in the positive mode were observed, indicating a molecular weight of 254.

### Test Example 1: Cytotoxicity Test

A cytotoxicity test was performed on the sample, which had been identified to be floridoside, by using the Cell Counting Kit-8 kit (DOJINDO, CK04-05).

Raw 264.7 cells were cultured at 1×10⁴ cells/well in a DMEM medium containing 10% FBS in a 96-well plate at 37°C in 5% CO₂ incubator for 24 hours, and then treated with a drug. After the cells were cultured at 37°C in the 5% CO₂ incubator for 24 hours, CCK-8 solution was added, followed by incubation at 37°C for 2 hours, and then the absorbance was measured at 450 nm. The results are shown in Table 1 below.

**TABLE 1**

| Concentration (ppm) | | Absorbance (nm) | | | AVERAGE | Standard deviation | Viability (%) |
|---|---|---|---|---|---|---|---|
| Floridoside | 1 | 2.726 | 2.589 | 2.466 | 2.594 | 0.106 | 83 |
| | 10 | 2.692 | 2.735 | 2.810 | 2.746 | 0.049 | 88 |
| | 100 | 2.676 | 2.790 | 2.810 | 2.759 | 0.059 | 88 |
| | 500 | 2.721 | 2.801 | 2.849 | 2.790 | 0.053 | 89 |
| | 1000 | 2.751 | 2.769 | 2.824 | 2.781 | 0.031 | 89 |
| con | | 3.113 | 3.1 | 3.182 | 3.132 | 0.036 | 100 |

As can be seen from Table 1, as the floridoside content increased, the viability was also improved. It can be identified from the results that floridoside had an excellent cell protective effect.

### Test Example 2: Patch Test

A primary patch test for human body application was performed on the sample.

Subjects of the test were determined for skin irritation by a dermatologist a total of 4 times (the date of a patch, the date of removal of the patch, and 24 hours and 120 hours after removal of the patch) during the test period.

The skin patch test was performed using the Van der Bend chamber (Van der Bend, Brielle, the Netherlands). For each of 31 subjects, the sample was applied between the shoulder blade and the waist line on the basis of the spin on the back by using the Van der Bend chamber.

A site of the test was washed with distilled water and dried, and then the sample was dissolved in 10% distilled water. Thereafter, 35 µl was dropped in the Van der Bend chamber, which was then put and fixed on the site of the test. The sample was patched on the skin for 48 hours, and after the removal of the patch, the site of the test was marked with a marking pen, and the site of the test was observed after 30 minutes, 24 hours, and 120 hours.

With respect to the skin reaction, the degrees of irritation were classified according to the evaluation criteria of the International Contact Dermatitis Research Group (ICDRG) as shown in Table 2 below.

**TABLE 2**

| Symbol | Score | Skin reaction evaluation criteria | |
|---|---|---|---|
| - | 0 | Negative reaction: No irritation | |
| ± | 0.5 | Doubtful or slight reaction: Slight irritation | Slight or faint erythema |
| + | 1 | Weak (non-vesicular) positive reaction: Weak irritation | Clear boundary lined but weak erythema, edema, and papule |
| ++ | 2 | Strong (vesicular) positive reaction: Medium irritation | Clear erythema, papule, and small blister |
| +++ | 3 | Extreme positive reaction: Strong irritation | Strong erythema and blister |

The mean score of the skin reaction was calculated by the skin reaction calculation formula, and then the presence or absence of irritation was determined according to the decision table of the skin patch test. The skin patch test results are shown in Table 3.

**TABLE 3**

| NO | | After 30 minutes | After 24 hours | After 120 hours |
|---|---|---|---|---|
| 1 | | - | - | - |
| 2 | | - | - | - |
| 3 | | - | - | - |
| 4 | | - | - | - |
| 5 | | - | - | - |
| 6 | | - | - | - |
| 7 | | - | - | - |
| 8 | | - | - | - |
| 9 | | - | - | - |
| 10 | | - | - | - |
| 11 | | - | - | - |
| 12 | | - | - | - |
| 13 | | - | - | - |
| 14 | | - | - | - |
| 15 | | - | - | - |
| 16 | | - | - | - |
| 17 | | - | - | - |
| 18 | | - | - | - |
| 19 | | - | - | - |
| 20 | | - | - | - |
| 21 | | - | - | - |
| 22 | | - | - | - |
| 23 | | - | - | - |
| 24 | | - | - | - |
| 25 | | - | - | - |
| 26 | | - | - | - |
| 27 | | - | - | - |
| 28 | | - | - | - |
| 29 | | - | - | - |
| 30 | | - | - | - |
| 31 | | - | - | - |
| Degree of reaction | ± | 0 | 0 | 0 |
| | + | 0 | 0 | 0 |
| | ++ | 0 | 0 | 0 |
| | +++ | 0 | 0 | 0 |
| Mean score | | 0.00 | | |
| Determination | | No irritation | | |

### Preparative Example 2: Synthesis of Glycoside Covalent Derivative of Floridoside and Nucleobase

A purine was bound to the floridoside prepared in Preparative Example 1 by solid phase peptide synthesis using Fmoc. As a result of ¹H-NMR spectrum and ¹³C-NMR spectrum analyses, a glycosidic covalent bond was formed between floridoside and the nucleobase as shown in FIG. 6.

### Preparative Example 3: Synthesis of glycoside covalent derivative of floridoside and nucleoside

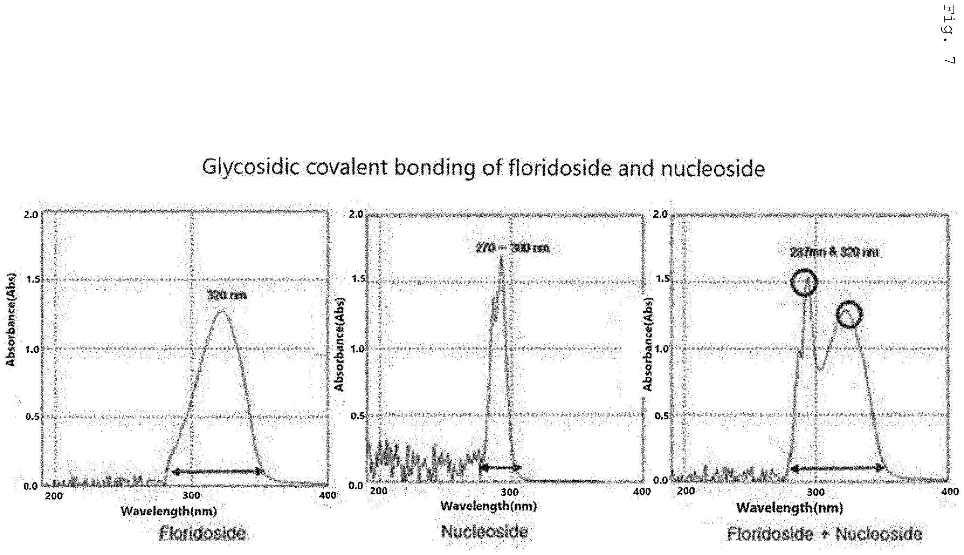

A ribonucleoside was bound to the floridoside prepared in Preparative Example 1 by solid phase peptide synthesis using Fmoc. As a result of ¹H-NMR spectrum and ¹³C-NMR spectrum analyses, a glycosidic covalent bond was formed between floridoside and the nucleoside as shown in FIG. 7.

### Preparative Example 4: Synthesis of glycoside covalent derivative of floridoside and amino acid

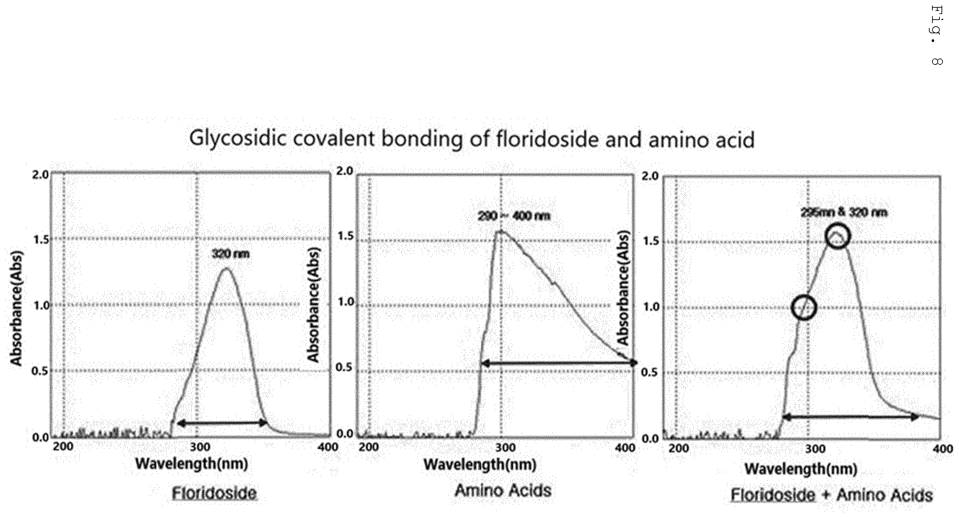

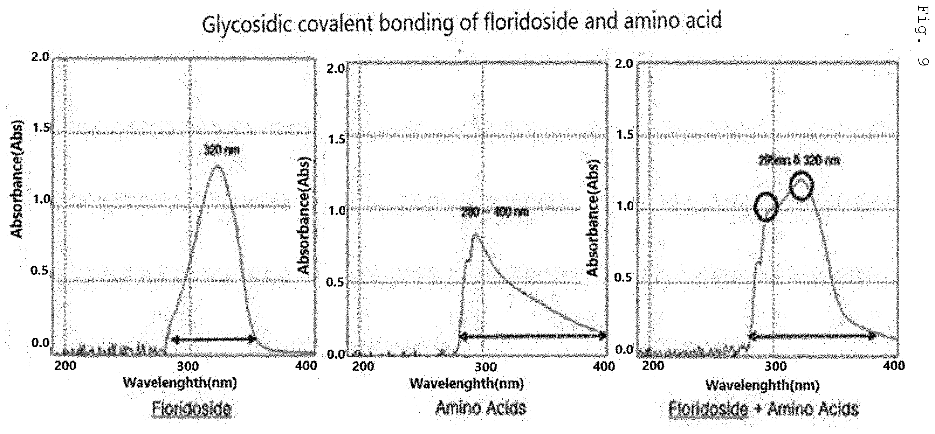

Alanine was bound to the floridoside prepared in Preparative Example 1 by solid phase peptide synthesis using Fmoc. As a result of ¹H-NMR spectrum and ¹³C-NMR spectrum analyses, a glycosidic covalent bond was formed between floridoside and the amino acid as shown in FIGS. 8 and 9.

### Example 1

The glycoside covalent derivative of floridoside and the nucleobase in Preparative Example 2 was diluted in distilled water to prepare a composition having a covalent derivative content of 25 wt% relative to 100 wt% of the total composition.

### Example 2

The glycoside covalent derivative of floridoside and the nucleoside in Preparative Example 3 was diluted in distilled water to prepare a composition having a covalent derivative content of 25 wt% relative to 100 wt% of the total composition.

### Example 3

The glycoside covalent derivative of floridoside and the amino acid in Preparative Example 4 was diluted in distilled water to prepare a composition having a covalent derivative content of 25 wt% relative to 100 wt% of the total composition.

### Test Example 3: In Vitro UVA Evaluation Test

To test UV blocking test of the compositions of Examples 1 to 3, the *in vitro* UVA evaluation test was performed using a UV spectrophotometer.

Each of the compositions (1.3 mg/cm²) of Examples 1 to 3 was well spread on the PMMA plate, dried for 15 minutes, and then placed into the SPF-290AS^{™} SPF Testing Analyzer System. The UV absorbance at 290-400 nm was measured for 8 points on the plate. The mean value was calculated by repeated measurements using three plates for each composition. SPF (UVB) and PA (UVA) measurement results are shown in Table 4.

**TABLE 4**

| | Number | SPF | PA | | Number | SPF | PA | | Number | SPF | PA |
|---|---|---|---|---|---|---|---|---|---|---|---|
| Example 1 | 1 | 5.3 | 4.1 | Example 2 | 1 | 3.4 | 2. 8 | Example 3 | 1 | 2.3 | 2.1 |
| | 2 | 5. 7 | 4.5 | | 2 | 3.7 | 3.2 | | 2 | 2.3 | 1.9 |
| | 3 | 5. 6 | 4.5 | | 3 | 3.4 | 2.9 | | 3 | 2.5 | 2.0 |
| | Mean | 5.6 | 4.4 | | Mean | 3.5 | 3.0 | | Mean | 2. 4 | 2.0 |
| | Standard Deviation | 0.2 | 0.2 | | Standard Deviation | 0.1 | 0.2 | | Standard Deviation | 0.1 | 0.1 |

As can be seen from Table 4 and FIGS. 6 to 9, the floridoside according to Examples 1 to 3 exhibited an enhanced UV blocking effect by forming a glycosidic covalent bond with a nucleobase, a nucleoside, or an amino acid.

## Claims

1. A UV blocking composition containing red algae-derived floridoside and an amine group-containing compound.

2. The composition of claim 1, wherein the red algae-derived floridoside includes red algae-derived glycerol galactoside, red algae-derived glycerol glucoside, or both thereof.

3. The composition of claim 2, wherein the red algae-derived glycerol galactoside is floridoside represented by chemical formula 1, or a salt or isomer thereof:

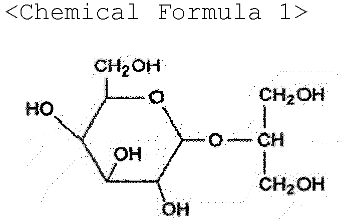

4. The composition of claim 2, wherein the red algae-derived glycerol galactoside is isofloridoside represented by chemical formula 2, or a salt or isomer thereof:

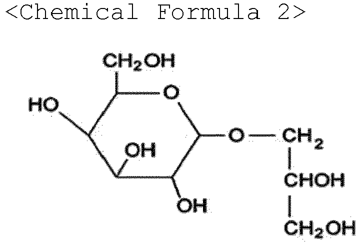

5. The composition of claim 1, wherein the amine group-containing compound is at least one selected from the group consisting of a nucleobase, a nucleoside, and an amino acid.

6. The composition of claim 1, wherein the red algae-derived floridoside and the amine group-containing compound form a covalent bond.

7. The composition of claim 1, wherein the red algae-derived floridoside is alkylated by the amine group-containing compound.

8. The composition of claim 5, wherein the nucleobase is at least one selected from adenine, guanine, cytosine, thymine, and uracil.

9. The composition of claim 5, wherein the nucleoside includes a ribonucleoside, a deoxyribonucleoside, or both thereof.

10. The composition of claim 1, wherein the red algae-derived floridoside and the amine group-containing compound form a glycoside covalent derivative.

11. A UV blocking cosmetic composition comprising the composition of any one of claims 1 to 10.

12. A UV blocking pharmaceutical composition comprising the composition of any one of claims 1 to 10.
